# EUROPÄISCHE PATENTSCHRIFT

(11) **EP 1 539 155 B1**
(45) Veröffentlichungstag und Bekanntmachung des Hinweises auf die Patenterteilung: **02.04.2008**
(21) Anmeldenummer: 03794750.4
(22) Anmeldetag: 11.09.2003
(51) Int. Cl.: A61K 31/4418, A61K 9/06

(54) **STABILE CREME-ZUBEREITUNGEN VON PHENYL-PYRIDONVERBINDUNGEN FÜR TOPISCHE ANWENDUNG**
STABLE CREAM PREPARATIONS OF PHENYL-PYRIDONE COMPOUNDS FOR TOPICAL APPLICATION
PREPARATIONS STABLES DE CREME A BASE DE COMPOSES DE PHENYL-PYRIDONE POUR APPLICATION TOPIQUE

(30) Priorität: 13.09.2002 CH 155602
(43) Veröffentlichungstag der Anmeldung: 15.06.2005
(73) Patentinhaber: Mepha AG, CH-4147 Aesch (CH)
(72) Erfinder: SCHEIWE, Max, Werner, 79689 Maulburg (DE); CETINKAYA, Yalcin, CH-4402 Frenkendorf (CH)
(74) Vertreter: Braun, André jr.
(86) Internationale Anmeldenummer: PCT/CH2003/000615
(87) Internationale Veröffentlichungsnummer: WO 2004/024152

(56) Entgegenhaltungen:
- WO-A-00/16775
- JUMAA M ET AL: "Parenteral emulsions stabilized with a mixture of phospholipids and PEG-660-12-hydroxy-stearate: evaluation of accelerated and long-term stability" EUROPEAN JOURNAL OF PHARMACEUTICS AND BIOPHARMACEUTICS, ELSEVIER SCIENCE PUBLISHERS B.V., AMSTERDAM, NL, Bd. 54, Nr. 2, September 2002 (2002-09), Seiten 207-212, XP004377365 ISSN: 0939-6411

## Beschreibung

Die vorliegende Erfindung betrifft neue stabile Creme-Zubereitungen für topische Anwendungen, welche als Wirkstoff mindestens eine gegebenenfalls substituierte Phenyl-Pyridonverbindung enthalten. Die erfindungsgemässen Zubereitungen zeichnen sich durch gute chemische und physikalische Stabilität aus und sind lagerstabil. Die erfindungsgemässen Creme-Zubereitungen eignen sich für die medizinische Behandlung von Hauterkrankungen, insbesondere solcher fibrotischer Natur.

Die Behandlung von Hauterkrankungen fibrotischer Natur mit Emulsionen, welche gegebenenfalls substituierte Phenyl-Pyridone enthalten, ist beispielsweise in US 5,310,562 und EP 0 383 591 beschrieben. So hat z.B. Pirfenidon [5-Methyl-1-Phenyl-2-(1H)-Pyridon] ein breites Anwendungsspektrum in der Behandlung und Prophylaxe von Gewebe- und Hauterkrankungen, wie z.B. fibröse Läsionen, Lungenfibrose, Fibrose der Prostata, Sklerosen, Keloide, Kollagenosen, Narbenfalten, postoperative Verwachsungen, Alzheimer-Krankheit, etc.

WO 97/41830 beschreibt die Verwendung von substituierten Pyridonen zur Heilung und Prophylaxe von fibrosen Läsionen in Form von unterschiedlichen Darreichungsformen, wie Kapseln, Tabletten, Pulvern, Granulaten, Sirups, injizierbaren Flüssigkeiten, Cremes, Salben, inhalierbaren-Flüssigkeiten, Augentropfen, Suppositorien und Pillen.

WO 99/47140 offenbart die Verwendung von gegebenenfalls substituierten Phenyl-Pyridonen, wie z.B. Pirfenidon, in topischen pharmazeutisch wirksamen Zubereitungen, wie Salben, Cremes oder Schäume.

WO 00/16775 offenbart Gele für die topische Verwendung, welche eine gegebenenfalls substituierte Phenyl-Pyridonverbindung, insbesondere Pirfenidon, enthalten, für die Behandlung und Prophylaxe von Hauterkrankungen fibrotischer Natur. In der Einleitung werden auch Cremes und Salben erwähnt.

Es besteht aber weiterhin ein grosser Bedarf an pharmazeutisch wirksamen stabilen Cremes, welche eine gegebenenfalls substituierte Pyridonverbindung enthalten, zur Behandlung von fibrotischen Läsionen der Haut. Cremes werden auf der geschädigten Haut besonders wohltuend und kühlend empfunden und in vielen Fällen Salben oder Gelen vorgezogen. Hydrophile Cremes verleihen ferner der Haut Feuchtigkeit und entfalten pflegende Eigenschaften. Cremes ziehen ausserdem meist vollständig in die Haut ein, wohingegen Gele auf der Hautoberfläche eintrocknen und zu einem Film führen, welcher in manchen Fällen als störend empfunden wird (Spannungsgefühl, kosmetische Beeinträchtigung durch schuppenähnliche Strukturen etc.).

Versuche haben jedoch gezeigt, dass Pyridonverbindungen, wie beispielsweise Pirfenidon, ungeeignet sind für die Verwendung in Emulsionen, da diese Pyridonverbindungen sich als Emulsionsbrecher verhalten, d.h. sie destabilisieren die Emulsion, in der sie enthalten sind. Wenn eine Emulsion "bricht", dann trennen sich die öligen und wässrigen Phasen und es kommt zur unerwünschten Koaleszenz, d.h. eine Art des Zusammenfliessens oder Gerinnens der Bestandteile. Dies führt oft zu einer Veränderung des pH-Wertes und unter Umständen sogar zur Auskristallisation des Wirkstoffs. Diese nachteilige Eigenschaft des Pirfenidons wird in den genannten Patenten und Patentanmeldungen nicht diskutiert und keine Lehre zur Behebung angeboten. Cremes werden lediglich als mögliche topische Anwendungsform erwähnt.

Cremes sind mehrphasige Arzneiformen, die halbfest sind. Sie sind sogenannte "nicht wegfliessende" Emulsionen bestehend aus einer lipophilen und einer hydrophilen wässrigen Phase. Sie können den Wirkstoff gelöst oder dispergiert in der wässrigen oder in der öligen Phase enthalten.

Um therapeutisch brauchbare Creme-Zubereitungen herzustellen, ist es unumgänglich, die Neigung des Pirfenidons zur Emulsionsbrechung zu überwinden, um somit die chemische und physikalische Stabilität sowie die Lagerstabilität seiner Emulsion zu gewährleisten. Die Stabilitätsdaten sind an der Zulassung von pharmazeutischen Produkten durch die Gesundheitsbehörden massgeblich beteiligt.

Typische Parameter der Stabilität sind die Homogenität der Formulierung, die Abwesenheit von Koaleszenz der Emulsionströpfchen (keine "Gerinnung"), praktisch konstante Viskosität, halbfeste Strukturen, die vollständige Auflösung des Wirkstoffs sowie keine spätere Auskristallisation des Wirkstoffs.

Ein weiteres Erfordernis an pharmazeutisch zulässigen Formulierungen ist, dass nur Hilfsstoffe verwendet werden, die pharmazeutisch akzeptabel sind und vorzugsweise in den Arzneibüchern beschrieben sind. Bei Hilfsstoffen, die in den Arzneibüchern nicht beschrieben sind, muss deren toxikologische Unbedenklichkeit durch meist kostspielige toxikologische Untersuchungen nachgewiesen werden. Ebenso muss nachgewiesen werden, dass die Sicherheit der Patienten, d.h. die Verträglichkeit und Wirksamkeit des Medikamentes in der therapeutischen Anwendung gewährleistet ist.

Untersuchungen haben gezeigt, dass standardmässige Hilfsstoff-Formulierungen, wie solche beispielsweise in der USP (United States Pharmacopoeia) beschrieben sind, sich nicht eignen für die Herstellung von pharmazeutisch akzeptablen Emulsionen für topische Anwendungen mit Pirfenidon als Wirkstoff.

Die Aufgabe der vorliegenden Erfindung ist daher, neue Creme-Zubereitungen mit Pyridonen als Wirkstoff zur Verfügung zu stellen, die ihre pharmazeutische Wirksamkeit behalten und gleichzeitig, selbst bei allfälliger Temperaturbelastung, chemisch und physikalisch stabil sind und welche eine gute Lagerstabilität aufweisen. Es wurde nun gefunden, dass Cremes mit der in weiteren angegebenen Formulierung überraschenderweise eine hervorragende Stabilität aufweisen.

Gegenstand der vorliegenden Erfindung ist eine pharmazeutische Creme-Zubereitung für topische Anwendung in Form einer Öl-in-Wasser (o/w) Emulsion, zur Behandlung und/oder Vorbeugung von Hauterkrankungen, welche dadurch gekennzeichnet ist, dass diese in der lipophilen Phase die folgenden Bestandteile enthält:
(i) als Wirkstoff eine gegebenenfalls substituierte 1-Phenyl-2-(1H)-Pyridonverbindung, oder ein pharmazeutisch annehmbares Salz davon,
(ii) Macrogol-15-hydroxystearat als oberflächenaktiven Solubilisator,
(iii) mindestens einen Emulgator ausgewählt aus Cetylstearylalkohol und Stearinsaure, sowie
(iv) gegebenenfalls weitere an sich bekannte Trägermaterialien und Additive ausgewählt aus der Gruppe enthaltend Triglyceride, Penetrationsverstärker, Konservierungsmittel und Antioxidationsmittel.

Die Öl-in-Wasser (o/w) Emulsion enthält vorzugsweise die ölige Phase in einem Bereich von etwa 20-80 Gew.-% und die wässerige Phase in einem Bereich von etwa 80-20 Gew.-%.

Bevorzugt ist die ölige Phase in einem Bereich von 24.1-84.1 Gew.-% und die wässerige Phase in einem Bereich von 75.9-15.9 Gew.-%; vorzugsweise ist die ölige Phase in einem Bereich von 37.2-65 Gew.-% und die wässerige Phase in einem Bereich von 35-62.8 Gew.-%, bezogen auf das Gesamtgewicht der erfindungsgemässen Zubereitung.

Die erfindungsgemässe Zubereitung bzw. Formulierung enthält den Wirkstoff [Komponente (i)] in der lipophilen Phase vorzugsweise in einer Menge von 0.5-9 Gew.-%, und vorzugsweise in einer Menge von 3-7 Gew.-%, bezogen auf das Gesamtgewicht der Zubereitung.

Die erfindungsgemässe Zubereitung bzw. Formulierung enthält den oberflächenaktiven Solubilisator (Lösungsvermittler) Macrogol-15-hydroxystearat [Komponente (ii)] vorzugsweise in einer Konzentration von 5-65 Gew.-%, und vorzugsweise in einer Konzentration von 10-45 Gew.-%, bezogen auf das Gesamtgewicht der Zubereitung.

Die erfindungsgemässe Zubereitung bzw. Formulierung enthält den Emulgator ausgewählt aus Cetylstearylalkohol und Stearinsäure [Komponente (iii)] vorzugsweise in einer Konzentration von 3-30 Gew.-%, und vorzugsweise in einer Konzentration von 5-12.5 Gew.-%, bezogen auf das Gesamtgewicht der Zubereitung.

Die Creme-Zubereitungen enthalten als Wirkstoff ein substituiertes Pyridon der allgemeinen Formel (I): 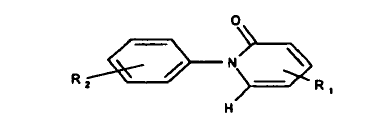 oder ein pharmazeutisch annehmbares Salz davon, worin R₁ und R₂ unabhängig voneinander (C₁-C₄) -Alkyl, Carboxyl (-COOH) oder -COOAlkyl(C₁-C₄) bedeuten, und R₂ zusätzlich auch Wasserstoff bedeuten kann.

R₁ und R₂ bedeuten als (C₁-C₄)-Alkyl unabhängig voneinander vorzugsweise Methyl, Ethyl, Propyl, Isopropyl, n-Butyl, sek.-Butyl oder t-Butyl. Bedeuten R₁ und/oder R₂ einen Rest -COOAlkyl(C₁-C₄), so hat darin der (C₁-C₄) - Alkylrest eine der vorgehend für R₁ und/oder R₂ genannten Bedeutungen.

Bevorzugt sind substituierte Pyridone der allgemeinen Formel (I), worin R₁ (C₁-C₄)-Alkyl und R₂ Wasserstoff oder (C₁-C₄)-Alkyl bedeuten. Besonders bevorzugt ist die Verbindung der Formel (I) worin R₁ Methyl und R₂ Wasserstoff bedeuten (Pirfenidon).

Als pharmazeutisch annehmbare Salze der Pyridonverbindungen der allgemeinen Formel (I) sind die für den Fachmann bekannten Salze anzusehen, wie z.B die Alkali- und Erdalkalisalze der mit Carboxyl substituierten Verbindung der Formel (I), vorzugsweise der Salze mit Natrium, Magnesium; oder der Salze der Verbindung der Formel (I), welche kein Carboxyl enthält, mit Oxalsäure, Bernsteinsäure, etc.

Als Beispiele für Vertreter der Pyridone der Formel (I), welche in den erfindungsgemässen Creme-Zubereitungen als Wirkstoff enthalten sein können, seien die folgenden bevorzugten Verbindungen genannt:
5-Methyl-1-p-Tolyl-2-(1H)-Pyridon
3-Methyl-1-Phenyl-2-(1H)-Pyridon
3-Ethyl-1-Phenyl-2-(1H)-Pyridon
4-Isopropyl-1- Phenyl-2-(1H)-Pyridon
5-Methyl-1-Phenyl-2-(1H)-Pyridon
3-Methyl-1-Carboxyphenyl-2-(1H)-Pyridon
5-Carboxy-1-Phenyl-2-(1H)-Pyridon
4-Carboxymethyl-1-Phenyl-2-(1H)-Pyridon
5-t-Butyl-1-(p-Carboxyethylphenyl)-2-(1H-)Pyridon.

Bevorzugt ist als Wirkstoff 5-Methyl-1-Phenyl-2-(1H)-Pyridon, genannt Pirfenidon.

Die substituierten Pyridone sind bekannte Verbindungen und können nach den üblichen, dem Fachmann bekannten, Techniken hergestellt werden, wie sie beispielsweise in US Patent 3,974,281 beschrieben sind.

Die erfindungsgemässen Cremes sind Öl-in-Wasser Emulsionen, die in der äusseren Phase wässrig sind.

Als oberflächenaktiver Solubilisator (Lösungsvermittler) eignet sich Solutol HS 15 (Macrogol-15-hydroxystearat von BASF, PEG-660-15-hydroxystearate).

Als Emulgatoren kommen die folgenden in Frage:
Cetylstearylalkohol, Stearinsäure.

Als Triglyceride kommen mittelkettige und hoch-molekulare Triglyceride in Frage. Mittelkettige Triglyceride sind Glycerinester der Fettsäuren mit nur 6-12 Kohlenstoffatomen, wie z.B. Capryl-Caprinsäuretriglycerid. Hochmolekulare Triglyceride sind Glycerinfettsäureester mit langkettigen Fettsäuren. Sie sind z.B. aus verschiedenen natürlichen Fetten hergestellte Triglyceridgemische. Bevorzugt werden mittelkettige Triglyceride eingesetzt, insbesondere Capryl-Caprinsäuretriglycerid.

Zu den geeigneten Eindringungsverstärker (penetration enhancers) zählen z.B. Isopropylmyristat, Ölsäure, Natriumlaurylsulfat oder 1,2-Propandiol. Bevorzugt ist 1,2-Propandiol.

Typische Beispiele für Konservierungsmittel sind Benzylbenzoate, Benzoesäure, Benzylalkohol, Benzalkoniumchlorid, N-Cetyl-N,N,N-Trimethylammoniumbromid (Cetrimid, Fa. Merck), Chlorhexidin, Chlorbutanol, Chlorcresol, Imidurea, die Parabene, wie Methyl-, Ethyl-, Propyl- oder Butylparaben, Natriummethylparaben, Natriumpropylparaben, Kaliumsorbat, Natriumbenzoat, Natriumpropionat, Phenol, Phenoxyethanol, Phenylethylalkohol, Phenylmercuriacetat, Phenylmercuriborat, Phenylmercurinitrate, Sorbinsäure oder Thiomersal (Natriumethylmercurithiosalicylat). Bevorzugt sind Methylparaben, Propylparaben sowie Natriummethylparaben und Natriumpropylparaben.

Beispiele für Antioxidantien sind Natriummetabisulfit, alpha-Tocopherol, Ascorbinsäure, Maleinsäure, Natriumascorbat, Ascorbylpalmitat, butyliertes Hydroxyanisol, butyliertes Hydroxytoluol, Fumarsäure oder Propylgallat. Bevorzugtes Antioxidant ist Natriummetabisulfit.

Als pH-regulierendes Mittel kommen z.B. Natriumhydroxid, Salzsäure, Puffersubstanzen, wie z.B. Natriumdihydrogenphosphat oder Dinatriumhydrogenphosphat, in Frage.

Die Creme-Zubereitungen können ferner weitere Hilfs- und Zusatzstoffe enthalten, wie z.B. Fettungsmittel, Lösungsmittel, Konsistenzgeber oder Hydrotrope, zur Verbesserung des Fliessverhaltens. Dabei können von den vorgehend angegebenen Additiven bzw. Zusatzstoffen jeweils einzelne oder auch mehrere Stoffe derselben Gruppe im Gemisch anwesend sein.

Als Fettungsmittel eignen sich z.B. Ölsäuredecylester, hydriertes Ricinusöl, leichtes Mineralöl, Mineralöl, Polyethylenglykol, Natriumlaurylsulfat.

Als Lösungsmittel kommen Maiskeimöl, Baumwollsaatöl, Erdnussöl, Sesamöl, Sojabohnenöl, Ethyloleat, Glycerin, Isopropylmyristat, Isopropylpalmitat, Polyethylenglykol oder Polypropylenglykol.

Als Konsistenzgeber eignen sich beispielsweise Cetylalkohol, Cetylesterwachs, hydriertes Ricinusöl, mikrokristalline Wachse, nichtionische Emulgatorwachse, Bienenwachs, Paraffin oder Stearylalkohol.

Geeignete Hydrotope sind Alkohole, wie beispielsweise Ethanol, Isopropylalkohol oder Polyole, wie z.B. Glycerin.

Typische Formulierungen der erfindungsgemässen Creme-Zubereitungen enthalten
(a) 3-7 Gew.-% Wirkstoff
(b) 3-30 Gew.-% Emulgator
(c) 5-65 Gew.-% oberflächenaktiver Solubilisator
(d) 5-30 Gew.-%Triglycerid
(e) 2-20 Gew.-% Penetrationsverstärker
(f) 2-20 Gew,-% Fettungsmittel
(g) 3-30 Gew.-% Konsistenzgeber
(h) 0,01-3 Gew.-% Konservierungsmittel
(i) 0,1-5 Gew.-% Antioxidationsmittel
(j) 1-50 Gew.-% Lösungsmittel
(k) gereinigte Wasser ad 100 Gew.-% (d.h.20-80 Gew.-% Wasser, insbesondere 15.9-75.9 Gew.-%)

Bevorzugte Creme-Zubereitungen der Erfindung enthalten
- 3-7 Gew.-% Wirkstoff
- 5-12,5 Gew.-% Cetylstearylalkohol
- 10-45 Gew.-% Macrogol-15-hydroxystearat
- 7-20 Gew.-% mittelkettiges Triglycerid
- 3-10 Gew.-% Propandiol
- 3-10 Gew,-% Ölsäuredecylester
- 5-12,5 Gew.-% Stearinsäure
- 0,02-3 Gew.-% Natriummethylparaben und Natriumpropylparaben
- 0,2-3 Gew.-% Natriummetabisulfit
- 1-50 Gew.-% Lösungsmittel
- Gereinigtes Wasser ad 100 Gew.-%.

Die Cremes werden dadurch zubereitet, dass man gleichzeitig, in separaten Vorrichtungen, die lipophilen Bestandteile zum Zusammenschmelzen bringt und die Schmelze auf 60-80°C erwärmt, und die wässrige Phase ebenfalls auf die gleiche Temperatur erwärmt. Anschliessend wird die wässrige Phase in die ölige Phase eingearbeitet, die Mischung homogen emulgiert und bis zur halbfesten Creme gerührt. Der pH-Wert wird vorzugsweise auf 5-7,5 eingestellt.

Die erfindungsgemässen topischen Creme-Zubereitungen eignen sich für die Behandlung oder Prophylaxe von Hauterkrankungen, wie sie in WO 00/16775 beschrieben sind. Sie sind besonders geeignet für die Behandlung und Prophylaxe von Hauterkrankungen fibrotischer Natur, wie z.B. fibröse Läsionen, multiple Warzen, Kontaktdermatitis, Keloide, sowie zur Förderung der Heilung von Brandwunden und zur postoperativen Wundversorgung etc.

Die erfindungsgemässen Zubereitungen liefern pharmazeutisch wirksame und kosmetisch ansprechende Cremes. Sie weisen gute chemische und physikalische Stabilität auf, sowohl nach der Herstellung, nach 3-6 Monaten als auch nach längerer Lagerung, so dass es weder zur Phasentrennung noch zur Auskristallisation des Wirkstoffs kommt.

Die folgenden Beispiele illustrieren die Erfindung.

### VEGLEICHSBEISPIELE

### Vergleichsbeispiel 1

Eine hydrophile Salbe gemäss USP 23 (United States Pharmacopoeia) wurde zubereitet:

| Hilfsstoffe | Mengenanteil |
|---|---|
| Polypropylenglykol | 12,0 g |
| Stearylalkohol | 25,0 g |
| Weisses Vaselin | 25,0 g |
| Methylparaben | 0,025 g |
| Propylparaben | 0,015 g |
| Natriumlaurylsulfat | 10,0 g |
| Gereinigtes Wasser | 27,9 g |

In diese Salbengrundlage wurde Pirfenidon, in den Mengen 3,5; 5,0 und 10 Gew-%, nach der konventionellen Technik, wie sie in USP 23 geschrieben ist, folgendermassen eingearbeitet:

Stearylalkohol und weisses Vaselin wurden im Damfbad geschmolzen und auf etwa 75°C erwärmt. Die übrigen Bestandteile, inklusive Pirfenidon, wurden hinzugefügt, nachdem sie in Wasser gelöst und ebenfalls auf 75°C erwärmt wurden. Das Gemisch wurde bis zum Festwerden gerührt. Die fertige Salbe wurde anschliessend in kleine Kunststofftuben mit Gewinde abgefüllt und mit einem Schraubendeckel verschlossen.

Die Stabilitätsvorprüfung zeigte, dass die Salbe physikalisch stabil war, bevor sie der kompletten Stabilitätsprüfung, inkl. der Ermittlung chemischen und physikalisch-chemischen Parametern, unterzogen wurde.

Nach 6 monatiger Lagerung unter Standardbedingungen (25°C ± 2°C, 59% rh ± 5%; "rh" steht für "relative humidity", relative Luftfeuchtigkeit) entstand in der Salbenzubereitung eine Phasentrennung, die Emulsion wurde durch Koaleszenz inhomogen (d.h. es kam zum Zusammenfliessen der Tröpfchen, "Gerinnen"). Ausserdem hat der Wirkstoff sich während der Lagerung auskristallisiert, und zwar in allen drei Konzentrationsproben mit jeweils 3,5 Gew.-%, 5 Gew.-% und 10 Gew.-% Pirfenidon. Ein Teil der Salben wies ferner einen so hohen Viskositätsverlust auf, dass sie sich verflüssigten.

Die nach USP 23 zubereitete Salbenformulierung mit Pirfenidon zeigt somit unzureichende Stabilität sowohl bezüglich der Salbenformulierung selbst als auch bezüglich des Wirkstoffes und ist für pharmazeutische Anwendung ungeeignet.

### Vergleichsversuch 2

Eine Creme wurde nach der folgenden Rezeptur zubereitet:

| Bestandteile | Mengenanteil |
|---|---|
| Pirfenidon | 5,0 g |
| Propylenglykol | 5,0 g |
| Ölsäuredecylester | 5,0 g |
| Mittelkettiges Triglycerid | 10,0 g |
| Diisopropyladipat | 5,0 g |
| Stearinsäure | 5,0 g |
| Cetylstearylalkohol | 5,0 g |
| Polyoxyethylen-40-stearat | 2,5 g |
| Sorbitanmonostearat | 2,5 g |
| Natriummethylparaben | 0,2 g |
| Natriumpropylparaben | 0,2 g |
| Gereinigtes Wasser | 54,6 g |

Die folgenden Bestandteile wurden im Dampfbad geschmolzen und unter langsamen Rühren auf 80°C erwärmt: Ölsäuredecylester, mittelkettiges Triglycerid, Diisopropyladipat, Stearinsäure, Cetylstearylalkohol, Polyoxyethylen-40-stearat und Sorbitan-monostearat. Die restlichen Bestandteile, inkl. Pirfenidon, wurden in Wasser gelöst und ebenfalls auf 80°C erwärmt. Die heisse wässrige Lösung wurde unter kräftigem Rühren zu der Schmelze gegeben und unter Rühren auf 30°C abgekühlt. Die fertige Creme wurde in Kunststofftuben mit Gewinde abgefüllt und mit Schraubendeckel verschlossen. Ein Teil wurde in Tuben abgefüllt. Stabilitätsprüfungen wurden, wie in Vergleichsbeispiel 1 beschrieben, durchgeführt.

Die Zubereitung war vor Beginn der Prüfungen homogen, d.h. stabil. Nach 6 Monaten Lagerung unter Standardbedingungen (25°C ± 2°C, 60% rh ± 5%), wies die Creme folgende Eigenschaften auf:
- Der Pirfenidongehalt der Probe blieb unverändert, d.h. sie blieb chemisch stabil.
- Die Probe enthielt auskristallisiertes Pirfenidon.

Die scharfkantigen Körner des auskristallisierten Wirkstoffs verursachten unannehmbare Kratzspuren beim Auftragen auf die Haut.

Die Creme-Zubereitung erwies sich durch diese Eigenschaft als ungeeignet für pharmazeutische Anwendungen und somit nicht zulassungsfähig bei den Gesundheitsbehörden.

### BEISPIEL 1

Eine erfindungsgemässe Creme wurde nach der folgenden Rezeptur zubereitet:

| Bestandteile | Mengenanteil |
|---|---|
| Pirfenidon | 500 g |
| Solutol HS 15 | 2500 g |
| Polypropylenglykol | 500 g |
| Ölsäuredecylester | 500 g |
| Mittelkettiges Triglycerid | 1000 g |
| Stearinsäure | 750 g |
| Cetylstearylalkohol | 750 g |
| Natriummethylparaben | 20 g |
| Natriumpropylparaben | 10 g |
| Gereinigtes Wasser | 3470 g |

Die Creme-Zubereitung wurde wie folgt hergestellt: Solutol HS 15 (Macrogol-15-hydroxystearat von BASF; ein nichtionischer Solubilisator) wurde im Wasserbad bei 75°C geschmolzen. Das Pirfenidon wurde unter Rühren in das geschmolzene Solutol HS 15 gegeben, bis eine klare Lösung entstand. Zu dieser Lösung wurden die Bestandteile Polypropylenglykol, Ölsäuredecylester, mittelkettiges Triglycerid, Stearinsäure und Cetylstearylalkohol gegeben, und das Rühren wurde bei 75°C fortgesetzt.

Natriummethylparaben und Natriumpropylparaben wurden in 3470 g gereinigtem Wasser bei 70°C gelöst. Die fetthaltige Schmelze und die wässrige Lösung wurden bei den genannten Temperaturen zusammen gemischt, kurz unter Vakuum gesetzt, und während 15 Minuten bei 70°C gerührt. Das Gemisch wurde an-schliessend unter Rühren auf 45°C abgekühlt, bei dieser Temperatur während 5 Minuten homogenisiert und weiter auf Raumtemperatur abgekühlt (≤ 25°C). Die erhaltene Creme wurde in Tuben abgefüllt. Es wurde eine Creme mit den folgenden Eigenschaften erhalten:

| | |
|---|---|
| Erscheinungsbild: | weiss bis farblos, homogen; keine Kristalle |
| pH (potentiometrisch): | 6,2 |
| Viskosität: | 38900 mPa.s |
| (Rotationstyp-Viskosimeter, Schergeschwindigkeit 11,8/s bei 20°C) | |
| Gehalt an Pirfenidon (HPLC): Wertes | 101,2 % des theoretischen |
| Gehalt an Verunreinigungen und Zersetzungsprodukten: (HPLC, 100% Methode) | < 0,1 % |

### Ergebnisse der Stabilitätsprüfungen:

Zu Beginn lag eine homogene Creme vor. Die eine Hälfte der Proben wurde bei Standardbedingungen (25°C ± 2°C, 60% rh ± 5%) gelagert, die zweite Hälfte bei 31°C ± 2°C, 70% rh ± 5%. Die Lagerung erfolgte während 6 Monaten.

Die Proben aus beiden Lagerbedingungen waren nach 6 Monaten immer noch frei von Kristallen. Der pH-Wert, die Viskosität, der Wirkstoffgehalt und die Zersetzungsprodukte wiesen nur die üblichen Abweichungen auf, die im Schwank-ungsbereich der Analysemethode liegen.

Die Zubereitung des Beispiels 1 erwies sich damit als stabil und kann als eine pharmazeutisch akzeptable Formulierung eingesetzt werden.

### BEISPIEL 2

Analog Beispiel 1 wurde die folgende Creme-Formulierung zubereitet:

| Bestandteile | Mengenanteil |
|---|---|
| Pirfenidon | 5,0 g |
| Solutol HS 15 | 10,0 g |
| Polypropylenglykol | 8,0 g |
| Ölsäuredecylester | 3,0 g |
| Mittelkettiges Triglycerid | 2,0 g |
| Stearylsäure | 10,0 g |
| Cetylstearylalkohol | 10,0 g |
| Natriummethylparaben | 0,2 g |
| Natriumpropylparaben | 0,2 g |
| Gereinigtes Wasser | 51,6 g |

Es wurde eine Creme mit den folgenden Eigenschaften erhalten:

| | |
|---|---|
| Erscheinungsbild: | weiss, homogen; keine |
| Kristalle | |
| pH (potentiometrisch): | 6,4 |
| viskosität: | 47020 mPa.s |
| (Rotationstyp-Viskosimeter, Schergeschwindigkeit 11,8/s bei 20°C) | |
| Gehalt an Pirfenidon (HPLC): | 100,8 % des theoretischen |
| Wertes | |
| Gehalt an Verunreinigungen und Zersetzungsprodukten: | < 0,1 % |
| (HPLC, 100% Methode) | |

### Ergebnisse der Stabilitätsprüfungen:

Zu Beginn lag eine homogene Creme vor. Die eine Hälfte der Proben wurde nun bei Standardbedingungen (25°C ± 2°C, 60% rh ± 5%) gelagert, die zweite Hälfte bei 31°C ± 2°C, 70% rh ± 5%. Die Lagerung erfolgte während 6 Monaten.

Die Proben aus beiden Lagerbedingungen waren nach 6 Monaten immer noch frei von Kristallen. Der pH-Wert, die Viskosität, der Wirkstoffgehalt und die Zersetzungsprodukte wiesen nur die üblichen Abweichungen auf, die im Schwank-ungsbereich der Analysemethode liegen.

Die Creme-Zubereitung des Beispiels 2 ist stabil und kann als eine pharmazeutisch akzeptable Formulierung eingesetzt werden.

### BEISPIEL 3

Analog Beispiel 1 wurde die folgende Creme-Formulierung zubereitet:

| Bestandteile | Mengenanteil |
|---|---|
| Pirfenidon | 5,0 g |
| Solutol HS 15 | 22,0 g |
| Polypropylenglykol | 3,0 g |
| Ölsäuredecylester | 12,0 g |
| Mittelkettiges Triglycerid | 9,0 g |
| Stearinsäure | 4,5 g |
| Cetylstearylalkohol | 4,5 g |
| Natriummethylparaben | 0,2 g |
| Natriumpropylparaben | 0,1 g |
| Natriummetabisulfit | 1,0 g |
| Gereinigtes Wasser | 39,7 g |

Bei der Herstellung der Creme wurde Natriummetabisulfit zusammen mit Natriummethylparaben und Natriumpropylparaben in Wasser bei 70°C gelöst. Ansonsten wurde wie in Beispiel 1 verfahren.

Es wurde eine Creme mit den folgenden Eigenschaften erhalten:

| | |
|---|---|
| Erscheinungsbild: | weiss, homogen; keine |
| Kristalle | |
| pH (potentiometrisch): | 6,3 |
| Viskosität: | 41256 mPa.s |
| (Rotationstyp-Viskosimeter, Schergeschwindigkeit 11,8/s bei 20°C) | |
| Gehalt an Pirfenidon (HPLC): | 99,7 % des theoretischen |
| Wertes | |
| Gehalt an Verunreinigungen und Zersetzungsprodukten: | < 0,1 % |
| (HPLC, 100% Methode) | |

### Ergebnisse der Stabilitätsprüfungen:

Zu Beginn lag eine homogene Creme vor. Die eine Hälfte der Proben wurde nun bei Standardbedingungen (25°C ± 2°C, 60% rh ± 5%) gelagert, die zweite Hälfte bei 31°C ± 2°C, 70% rh ± 5%. Die Lagerung erfolgte während 6 Monaten.

Die Proben aus beiden Lagerbedingungen waren nach 6 Monaten immer noch frei von Kristallen. Der pH-Wert, die Viskosität, der Wirkstoffgehalt und die Zersetzungsprodukte wiesen nur die üblichen Abweichungen auf, die im Schwankungsbereich der Analysemethode liegen.

Die Creme-Zubereitung aus Beispiel 3 ist stabil und für pharmazeutische Anwendung geeignet.

## Patentansprüche

1. Pharmazeutische Creme-Zubereitung für topische Anwendung in Form einer Öl-in-Wasser Emulsion, zur Behandlung und/oder Vorbeugung von Hauterkrankungen, **dadurch gekennzeichnet, dass** diese in der lipophilen Phase die folgenden Bestandteile enthält:
(i) als Wirkstoff eine gegebenenfalls substituierte 1-Phenyl-2-(1H)-Pyridonverbindung, oder ein pharmazeutisch annehmbares Salz davon,
(ii) Macrogol-15-hydroxystearat als oberflächenaktiven Solubilisator,
(iii) mindestens einen Emulgator, ausgewählt aus Cetyl-stearylalkohol und Stearinsäure, sowie
(iv) gegebenenfalls weitere an sich bekannte Trägermaterialien und Additive ausgewählt aus der Gruppe enthaltend Triglyceride, Penetrationsverstärker, Konservierungsmittel und Antioxidationsmittel.

2. Zubereitung nach Anspruch 1, **dadurch gekennzeichnet, dass** diese die ölige Phase in einem Bereich von 20-80 Gew.-% und die wässerige Phase in einem Bereich von 80-20 Gew.-%, bezogen auf das Gesamtgewicht der erfindungsgemässen Zubereitung, enthält.

3. Zubereitung nach Anspruch 1, **dadurch gekennzeichnet, dass** diese die ölige Phase in einem Bereich von 24.1-84.1 Gew.-% und die wässerige Phase in einem Bereich von 75.9-15.9 Gew.-%; vorzugsweise die ölige Phase in einem Bereich von 37.2-65 Gew.-% und die wässerige Phase in einem Bereich von 35-62.8 Gew.-%, bezogen auf das Gesamtgewicht der erfindungsgemässen Zubereitung, enthält.

4. Zubereitung nach einem der Ansprüche 1-3, **dadurch gekennzeichnet, dass** diese den Wirkstoff in einer Menge von 0.5-9 Gew.-%, und vorzugsweise in einer Menge von 3-7 Gew.-%, bezogen auf das Gesamtgewicht der Zubereitung, enthält.

5. Zubereitung nach einem der Ansprüche 1-4, **dadurch gekennzeichnet, dass** diese den oberflächenaktiven Solubilisator in einer Konzentration von 5-65 Gew.-%, und vorzugsweise in einer Konzentration von 10-45 Gew.-%, bezogen auf das Gesamtgewicht der Zubereitung, enthält.

6. Zubereitung nach einem der Ansprüche 1-5, **dadurch gekennzeichnet, dass** diese den Emulgator in einer Konzentration von 3-30 Gew.-%, und vorzugsweise in einer Konzentration von 5-12.5 Gew.-%, bezogen auf das Gesamtgewicht der Zubereitung, enthält.

7. Zubereitung nach einem der Ansprüche 1-6, **dadurch gekennzeichnet, dass** diese als Wirkstoff ein substituiertes Pyridon der allgemeinen Formel (I): 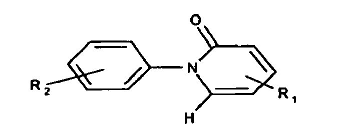 oder ein pharmazeutisch annehmbares Salz davon, enthält, worin R₁ und R₂ unabhängig voneinander (C₁-C₄)-Alkyl, Carboxyl (-COOH) oder -COOAlkyl(C₁-C₄) bedeuten, und R₂ zusätzlich auch Wasserstoff bedeutet.

8. Zubereitung nach Anspruch 7, **dadurch gekennzeichnet, dass** R₁ und R₂ als (C₁-C₄)-Alkyl unabhängig voneinander Methyl, Ethyl, Propyl, Isopropyl, n-Butyl, sek.-Butyl oder t-Butyl bedeuten und soweit R₁ und/oder R₂ einen Rest -COOAlkyl (C₁-C₄) bedeuten, darin der (C₁-C₄)-Alkylrest eine der vorgehend für R₁ und/oder R₂ genannten Bedeutungen hat.

9. Zubereitung nach Anspruch 7, **dadurch gekennzeichnet, dass** diese als Wirkstoff eine Verbindung der Formel (I) enthält, worin R₁ (C₁-C₄)-Alkyl und R₂ Wasserstoff oder (C₁-C₄)-Alkyl, vorzugsweise worin R₁ Methyl und R₂ Wasserstoff bedeuten.

10. Zubereitung nach einem der Ansprüche 1-9, **dadurch gekennzeichnet, dass** der Wirkstoff als pharmazeutisch annehmbares Salz als Alkali- und Erdalkalisalz der mit Carboxyl substituierten Verbindung der Formel (I), vorzugsweise als Natriumsalz oder Magnesiumsalz; oder als Salz der Verbindung der Formel (I), welche kein Carboxyl enthält, mit Oxalsäure oder Bernsteinsäure.

11. Zubereitung nach einem der Ansprüche 1-10, **dadurch gekennzeichnet, dass** als Wirkstoff eine der folgenden Verbindungen enthält:
5-Methyl-1-p-Tolyl-2-(1H)-Pyridon
3-Methyl-1-Phenyl-2-(1H)-Pyridon
3-Ethyl-1-Phenyl-2-(1H)-Pyridon
4-Isopropyl-1-Phenyl-2-(1H)-Pyridon
5-Methyl-1-Phenyl-2-(1H)-Pyridon
3-Methyl-1-Carboxyphenyl-2-(1H)-Pyridon
5-Carboxy-1-Phenyl-2-(1H)-Pyridon
4-Carboxymethyl-1-Phenyl-2-(1H)-Pyridon
5-t-Butyl-1-(p-Carboxyethylphenyl)-2-(1H-)Pyridon.

12. Zubereitung nach einem der Ansprüche 1-11, **dadurch gekennzeichnet, dass** das Triglycerid ausgewählt ist aus der Gruppe enthaltend mittelkettige und hochmolekulare Triglyceride, vorzugsweise mittelkettige Triglyceride als Glycerinester der Fettsäuren mit 6-12 Kohlenstoffatomen, vorzugsweise Capryl-Caprinsäuretriglycerid.

13. Zubereitung nach einem der Ansprüche 1-12, **dadurch gekennzeichnet, dass** der Eindringungsverstärker ausgewählt ist aus der Gruppe enthaltend Isopropylmyristat, Ölsäure, Natriumlaurylsulfat oder 1,2-Propandiol, vorzugsweise 1,2-Propandiol.

14. Zubereitung nach einem der Ansprüche 1-13, **dadurch gekennzeichnet, dass** diese im weiteren Fettungsmittel, Lösungsmittel, Konsistenzgeber und/oder Hydrotrope enthalten.

15. Zubereitung nach einem der Ansprüche 1-14, **dadurch gekennzeichnet, dass** diese die folgenden Komponenten enthält:
(a) 3-7 Gew.-% Wirkstoff
(b) 3-30 Gew.-% Emulgator
(c) 5-65 Gew.-% oberflächenaktiver Solubilisator
(d) 5-30 Gew.-%Triglycerid
(e) 2-20 Gew.-% Penetrationsverstärker
(f) 2-20 Gew,-% Fettungsmittel
(g) 3-30 Gew.-% Konsistenzgeber
(h) 0,01-3 Gew.-% Konservierungsmittel
(i) 0,1-5 Gew.-% Antioxidationsmittel
(k) 1-50 Gew.-% Lösungsmittel
(l) gereinigtes Wasser ad 100 Gew.-% d.h. 20-80 Gew.-% Wasser, insbesondere 15.9-75.9 Gew.-%.

16. Zubereitung nach einem der Ansprüche 1-15, **dadurch gekennzeichnet, dass** die folgenden Komponenten enthält:
- 3-7 Gew.-% Wirkstoff
- 5-12,5 Gew.-% Cetylstearylalkohol
- 10-45 Gew.-% Macrogol-15-hydroxystearat
- 7-20 Gew.-% mittelkettiges Triglycerid
- 3-10 Gew.-% Propandiol
- 3-10 Gew,-% Ölsäuredecylester
- 5-12,5 Gew.-% Stearinsäure
- 0,02-3 Gew.-% Natriummethylparaben und Natriumpropylparaben
- 0,2-3 Gew.-% Natriummetabisulfit
- 1-50 Gew.-% Lösungsmittel
- Gereinigtes Wasser ad 100 Gew.-%.

17. Zubereitung nach einem der Ansprüche 1-16 enthaltend Pirfenidon, Cetylstearylalkohol, Macrogol-15-hydroxystearat, mittelkettiges Triglycerid, Polypropylenglykol, Ölsäure-decylester, Stearinsäure, Natriummethylparaben, Natrium-propylparaben und Wasser.

18. Verfahren zur Herstellung einer Zubereitung nach einem der Ansprüche 1-17, **dadurch gekennzeichnet, dass** man in separaten Vorrichtungen, die lipophilen Bestandteile zum Zusammenschmelzen bringt und die Schmelze auf 60-80°C erwärmt, und die wässrige Phase auf dieselbe Temperatur erwärmt; anschliessend die wässrige Phase in die ölige Phase eingearbeitet, die Mischung homogen emulgiert und bis zur halbfesten Creme rührt, wobei der pH-Wert gegebenfalls auf 5-7,5 eingestellt wird.

19. Verwendung der Zubereitung nach einem der Ansprüche 1-18 zur Herstellung einer Creme für die topische Behandlung oder Prophylaxe von Hauterkrankungen, vorzugsweise für die Behandlung und Prophylaxe von Hauterkrankungen fibrotischer Natur, insbesondere fibröse Läsionen, multiple Warzen, Kontaktdermatitis, Keloide, sowie zur Förderung der Heilung von Brandwunden und zur postoperativen Wundversorgung.

## Claims

1. Pharmaceutical cream preparation for topical application in the form of an oil-in-water emulsion, for the treatment and/or prevention of skin diseases, **characterized in that** said preparation comprises the following constituents in the lipophilic phase:
(i) as the active ingredient, an optionally substituted 1-phenyl-2-(1H)-pyridone compound or a pharmaceutically acceptable salt thereof,
(ii) macrogol-15-hydroxystearate as surface-active solubilizer,
(iii)at least one emulsifier selected from cetyl stearyl alcohol and stearic acid, and
(iv) optionally other excipients and additives known per se and selected from the group comprising triglycerides, penetration enhancers, preservatives and antioxidants.

2. Preparation according to claim 1, **characterized in that** it comprises the oily phase in a range from 20 to 80% by weight and the aqueous phase in a range from 80 to 20% by weight, based on the total weight of the preparation according to the invention.

3. Preparation according to claim 1, **characterized in that** it comprises the oily phase in a range from 24.1 to 84.1% by weight and the aqueous phase in a range from 75.9 to 15.9% by weight; preferably it comprises the oily phase in a range from 37.2 to 65% by weight and the aqueous phase in a range from 35 to 62.8% by weight, based on the total weight of the preparation according to the invention.

4. Preparation according to any of claims 1 to 3, **characterized in that** it comprises the active ingredient in an amount of 0.5-9% by weight and preferably in an amount of 3 to 7% by weight, based on the total weight of the preparation.

5. Preparation according to any of claims 1-4, **characterized in that** it comprises the surface-active solubilizer in a concentration of 5-65% by weight and preferably in a concentration of 10 to 45% by weight, based on the total weight of the preparation.

6. Preparation according to any of claims 1 to 5, **characterized in that** it comprises the emulsifier in a concentration of 3-30% by weight, and preferably in a concentration of 5 to 12.5% by weight, based on the total weight of the preparation.

7. Preparation according to any of claims 1 to 6, **characterized in that** it comprises as the active ingredient a substituted pyridone of general formula (I): 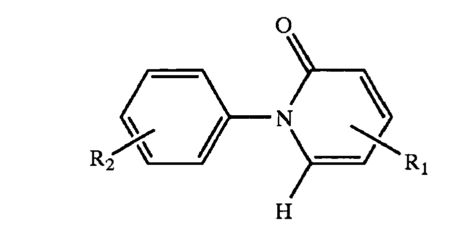 or a pharmaceutically acceptable salt thereof, in which R₁ and R₂ independently of one another are (C₁-C₄) alkyl, carboxyl (-COOH) or -COOalkyl (C₁-C₄) and R₂ is additionally also hydrogen.

8. Preparation according to claim 7, **characterized in that** R₁ and R₂ as (C₁-C₄)alkyl are independently of one another methyl, ethyl, propyl, isopropyl, n-butyl, sec-butyl or t-butyl and when R₁ and/or R₂ are a -COOalkyl (C₁-C₄) radical, the (C₁-C₄) -alkyl radical therein has one of the meanings mentioned above for R₁ and/or R₂.

9. Preparation according to claim 7, **characterized in that** it contains as the active ingredient a compound of formula (I) in which R₁ is (C₁-C₄) alkyl and R₂ is hydrogen or (C₁-C₄) alkyl, preferably in which R₁ is methyl and R₂ is hydrogen.

10. Preparation according to any of claims 1 to 9, **characterized in that** the active ingredient as a pharmaceutically acceptable salt is present as an alkali metal or alkaline earth metal salt of the carboxyl-substituted compound of formula (I), preferably as a sodium salt or magnesium salt; or as a salt of the compound of formula (I) which does not contain a carboxyl, with oxalic acid or succinic acid.

11. Preparation according to any of claims 1 to 10, **characterized in that** it comprises one of the following compounds as the active ingredient:
5-methyl-1-p-tolyl-2-(1H)-pyridone
3-methyl-1-phenyl-2-(1H)-pyridone
3-ethyl-1-phenyl-2-(1H)-pyridone
4-isopropyl-1-phenyl-2-(1H)-pyridone
5-methyl-1-phenyl-2-(1H)-pyridone
3-methyl-1-carboxyphenyl-2-(1H)-pyridone
5-carboxy-1-phenyl-2-(1H)-pyridone
4-carboxymethyl-1-phenyl-2-(1H)-pyridone
5-t-butyl-1-(p-carboxyethylphenyl)-2-(1H)-pyridone.

12. Preparation according to any of claims 1-11, **characterized in that** the triglyceride is selected from the group containing medium-chain and high molecular weight triglycerides, preferably medium-chain triglycerides as glycerol esters of the fatty acids having 6-12 carbon atoms, preferably caprylic/capric acid triglyceride.

13. Preparation according to any of claims 1 to 12, **characterized in that** the penetration enhancer is selected from the group consisting of isopropyl myristate, oleic acid, sodium laurylsulfate and 1,2-propanediol.

14. Preparation according to any of claims 1 to 13, **characterized in that** it also comprises superfatting agents, solvents, consistency regulators and/or hydrotropic agents.

15. Preparation according to any of claims 1 to 14, **characterized in that** it comprises the following components:
(a) 3-7% by weight of active ingredient
(b) 3-30% by weight of emulsifier
(c) 5-65% by weight of surface-active solubilizer
(d) 5-30% by weight of triglyceride
(e) 2-20% by weight of penetration enhancer
(f) 2-20% by weight of superfatting agent
(g) 3-30% by weight of consistency regulator
(h) 0.01-3% by weight of preservative
(i) 0.1-5% by weight of antioxidant
(k) 1-50% by weight of solvent
(l) purified water ad 100% by weight, i.e. 20 to 80% by weight of water, in particular 15.9 to 75.9% by weight.

16. Preparation according to any of claims 1 to 15, **characterized in that** it comprises the following components:
- 3-7% by weight of active ingredient
- 5-12.5% by weight of cetylstearyl alcohol
- 10-45% by weight of macrogol 15-hydroxystearate
- 7-20% by weight of medium-chain triglyceride
- 3-10% by weight of propanediol
- 3-10% by weight of decyl oleate
- 5-12.5% by weight of stearic acid
- 0.02-3% by weight of sodium methylparaben and sodium propylparaben
- 0.2-3% by weight of sodium metabisulfite
- 1-50% by weight of solvent
- purified water ad 100% by weight.

17. Preparation according to any of claims 1-16 comprising pirfenidone, cetyl stearyl alcohol, macrogol-15-hydroxystearate, medium-chain triglyceride, polypropylene glycol, decyl oleate, stearic acid, sodium methylparaben, sodium propylparaben and water.

18. Process for the production of a preparation according to any of claims 1 to 17, **characterized in that** the lipophilic constituents are melted together and the melt is heated to 60-80°C in separate apparatuses, and the aqueous phase is heated to the same temperature; the aqueous phase is then incorporated into the oily phase and the mixture is emulsified until homogeneous and stirred until it forms a semisolid cream, the pH optionally being adjusted to 5-7.5.

19. Use of the preparation according to any of claims 1 to 18 in the manufacture of a cream for the topical treatment or prophylaxis of skin diseases, preferably for the treatment and prophylaxis of skin diseases of a fibrotic nature, in particular fibrous lesions, multiple warts, contact dermatitis and keloids or for promoting the healing of burns or for post-operative wound care.

## Revendications

1. Préparation de crème pharmaceutique pour l'application topique sous forme d'une émulsion huile dans eau, pour le traitement et/ou la prévention de maladie de la peau, **caractérisée en ce que** celle-ci comprend dans la phase lipophile les composants suivants :
(i) comme substance active un composé de 1-phényl-2-(1 H)-pyridone optionnellement substitué ou un sel pharmaceutiquement acceptable de celui-ci,
(ii) du macrogol-15-hydroxystéarate en tant que solubilisateur tensioactif,
(iii) au moins un émulsifiant, choisi parmi alcool cétyl stéarylique et acide stéarique, ainsi que
(iv) optionnellement d'autres substances porteuses et additifs connus en soi, choisis dans le groupe comprenant triglycérides, activateurs de pénétration, conservateurs et antioxydants.

2. Préparation selon la revendication 1, **caractérisée en ce que** celle-ci comprend la phase huileuse dans un ordre de 20 - 80 % en poids et la phase aqueuse dans un ordre de 80 - 20 % en poids, par rapport au poids total de la préparation selon l'invention.

3. Préparation selon la revendication 1, **caractérisée en ce que** celle-ci comprend la phase huileuse dans un ordre de 24.1 - 84.1 % en poids et la phase aqueuse dans un ordre de 75.9 - 15.9 % en poids ; de préférence la phase huileuse dans un ordre de 37.2 - 65 % en poids et la phase aqueuse dans un ordre de 35 - 62.8 % en poids, par rapport au poids total de la préparation selon l'invention.

4. Préparation selon l'une quelconque des revendications 1 à 3, **caractérisée en ce que** celle-ci comprend la substance active en une quantité de 0.5 - 9 % en poids, de préférence en une quantité de 3 - 7 % en poids, par rapport au poids total de la préparation.

5. Préparation selon l'une quelconque des revendications 1 à 4, **caractérisée en ce que** celle-ci comprend le solubilisateur tensioactif en une concentration de 5 - 65 % en poids, et de préférence en une concentration de 10 - 45 % en poids, par rapport au poids total de la préparation.

6. Préparation selon l'une quelconque des revendications 1 à 5, **caractérisée en ce que** celle-ci comprend l'émulsifiant en une concentration de 3 - 30 % en poids, et de préférence en une concentration de 5 -12.5 % en poids, par rapport au poids total de la préparation.

7. Préparation selon l'une quelconque des revendications 1 à 6, **caractérisée en ce que** celle-ci comprend en tant que substance active une pyridone substituée ayant la formule générale (I) : 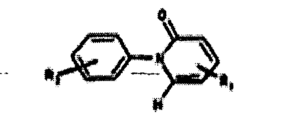 ou un sel pharmaceutiquement acceptable de celle-ci, dans laquelle R₁ et R₂ indépendant l'un de l'autre représentent de l'alkyle (C₁-C₄), carboxyle (-COOH) ou -COOalkyle (C₁-C₄), et R₂ représente en plus également hydrogène.

8. Préparation selon la revendication 7, **caractérisée en ce que** R₁ et R₂ en tant qu'alkyle (C₁-C₄) représentent, indépendant l'un de l'autre, méthyle, éthyle, propyle, isopropyle, n-butyle, sec-butyle ou t-butyle et si R₁ et/ou R₂ représentent un reste de -COOalkyle (C₁-C₄), alors le reste de l'alkyle (C₁-C₄) y a une des significations mentionnées précédemment pour R₁ et/ou R₂.

9. Préparation selon la revendication 7, **caractérisée en ce que** celle-ci comprend comme substance active un composé ayant la formule (I), dans laquelle R₁ représente de l'alkyle (C₁-C₄) et R₂ de l'hydrogène ou de l'alkyle (C₁-C₄), de préférence dans laquelle R₁ représente du méthyle et R₂ de l'hydrogène.

10. Préparation selon l'une quelconque des revendications 1 à 9, **caractérisée en ce que** la substance active est un sel pharmaceutiquement acceptable sous forme de sel alcalin ou sel alcalino-terreux du composé ayant la formule (I) substitué avec un carboxyle, de préférence du sel de sodium ou du sel de magnésium ; ou sous forme de sel du composé ayant la formule (I) qui ne comprend pas de carboxyle, avec de l'acide oxalique ou de l'acide succinique.

11. Préparation selon l'une quelconque des revendications 1 à 10, **caractérisée en ce que** celle-ci comprend comme substance active un composé parmi les suivants :
5-méthyl-1-p-tolyl-2-(1H)-pyridone
3-méthyl-1-phényl-2-(1H)-pyridone
3-éthyl-1-phényl-2-(1H)-pyridone
4-isopropyl-1-phényl-2-(H)-pyridone
5-méthyl-1-phényl-2-(1H)-pyridone
3-méthyl-1-carboxyphényl-2-(1H)-pyridone
5-carboxy-1-phényl-2-(1H)-pyridone
5-carboxyméthyl-1-phényl-2-(1H)-pyridone
5-t-butyle-1-(p-carboxyéthylphényl)-2-(1H)-pyridone.

12. Préparation selon l'une quelconque des revendications 1 à 11, **caractérisée en ce que** le triglycéride est choisi dans le groupe comprenant des triglycérides à chaînes moyennes et de poids moléculaire élevé, de préférence des triglycérides à chaînes moyennes sous forme d'ester de glycérine des acides gras avec 6 à 12 atomes carboniques, de préférence du triglycéride capryle - acide caprique.

13. Préparation selon l'une quelconque des revendications 1 à 12, **caractérisée en ce que** l'activateur de pénétration est choisi dans le groupe comprenant isopropylmyristate, acide oléique, sulfate laurique de sodium ou 1,2-propandiol, de préférence 1,2-propandiol.

14. Préparation selon l'une quelconque des revendications 1 à 13, **caractérisée en ce que** celle-ci comprend en plus des agents graissants, des solvants, des donneurs de consistance, et/ou des hydrotropes.

15. Préparation selon l'une quelconque des revendications 1 à 14, **caractérisée en ce que** celle-ci comprend les composants suivants :
(a) 3 - 7 % en poids de substance active
(b) 3 - 30 % en poids d'émulsifiant
(c) 5 - 65 % en poids de solubilisateur tensioactif
(d) 5 - 30 % en poids de triglycéride
(e) 2 - 20 % en poids d'activateur de pénétration
(f) 2 - 20 % en poids d'agent graissant
(g) 3 - 30 % en poids de donneur de consistance
(h) 0.01 - 3 % en poids de conservateur
(i) 0.1 - 5 % en poids d'antioxydant
(k) 1 - 50 % en poids de solvant
(l) de l'eau purifiée ad 100 % en poids, c'est-à-dire 20 - 80 % en poids d'eau, en particulier 15.9 - 75.9 % en poids.

16. Préparation selon l'une quelconque des revendications 1 à 15, **caractérisée en ce que** celle-ci comprend les composants suivants :
- 3 - 7 % en poids de substance active
- 5 - 12.5 % en poids d'alcool cétyl stéarylique
- 10 - 45 % en poids de macrogol-15-hydroxystéarate
- 7 - 20 % en poids de triglycéride à chaînes moyennes
- 3 -10 % en poids de propandiol
- 3 - 10 % en poids d'décylester d'acide oléique
- 5 -12.5 % en poids d'acide stéarique
- 0.02 - 3 % en poids de méthylparabène de sodium et de propylparabène de sodium
- 0.2 - 3 % en poids de métabisulfite de sodium
- 1 - 50 % en poids de solvant
- de l'eau purifiée ad 100 % en poids.

17. Préparation selon l'une quelconque des revendications 1 à 16, comprenant pirfénidone, alcool cétyl stéarylique, macrogol-15-hydroxystéarate, triglycéride à chaînes moyennes, polypropylèneglycol, décylester d'acide oléique, acide stéarique, méthylparabène de sodium, propylparabène de sodium et de l'eau.

18. Procédé pour la fabrication d'une préparation selon l'une quelconque des revendications 1 à 17, **caractérisé en ce que** les composants lipophiles sont fondus dans des dispositifs séparés et la masse fondue est chauffée à 60 - 80°C, et la phase aqueuse est chauffée à la même température ; ensuite la phase aqueuse est travaillée dans la phase huileuse, le mélange est émulsifié de manière homogène et tourné en une crème semi-solide, où la valeur pH est optionnellement réglée à 5 - 7.5.

19. Utilisation de la préparation selon l'une quelconque des revendications 1 à 18 pour la fabrication d'une crème pour le traitement topique ou la prophylaxie de maladies de la peau, de préférence pour le traitement et la prophylaxie de maladies de la peau de nature fibrotique, en particulier des lésions fibreuses, verrues multiples, dermatites de contact, chéloïdes, de même que pour stimuler la guérison d'une brûlure et pour les soins postopératoires de cicatrices.
